# EUROPEAN PATENT APPLICATION

(11) **EP 3 427 643 A1**
(43) Date of publication of application: **16.01.2019**
(21) Application number: 18182561.3
(22) Date of filing: 10.07.2018
(51) Int. Cl.: A61B 5/00, A61B 5/0402

(54) **EMBEDDING VISUAL INFORMATION INTO ECG SIGNAL IN REAL TIME**

(30) Priority: 11.07.2017 US 201715646285
(71) Applicant: Biosense Webster (Israel) Ltd., Yokneam 2066717 (IL)
(72) Inventor: AUERBACH, Shmuel, 2066717 Yokneam (IL); ZIGELMAN, Gil, 2066717 Yokneam (IL); GALKIN, Maxim, 2066717 Yokneam (IL)
(74) Representative: Small, Gary James

(57) **Abstract**

Embodiments include methods, systems, and apparatuses for generating an enhanced electrocardiograph (ECG) that includes indicators of values of different types of supplemental information embedded into a trace of measured electrical potentials. More specifically, embodiments may include collecting first data samples of electrical potentials produced by a heart at a sequence of sampling times, and processing the data to calculate supplemental information over a number of sampling times. Based on the first data samples and the supplemental information, a trace of the electrical potentials collected at the sampling times is presented. The trace may have one or more embedded indicators of the supplemental information that vary responsively to the first data samples collected at each of the sampling times.

## Description

### SUMMARY

Embodiments may include methods, systems, and apparatuses for generating an enhanced electrocardiograph (ECG) that may include one or more indicators of values of different types of ancillary data embedded into a trace of measured electrical potentials. For example, first data samples of electrical potentials are produced by a heart at a sequence of sampling times, wherein the first data samples are collected from one or more body surface electrodes, intracardiac electrodes, or both. Supplemental information may be generated based on at least a difference in the first data samples gathered over a number of the sampling times. Based on the first data samples, a trace of the electrical potentials collected at the sampling times may be generated. The trace may include a line chart. One or more indicators may be embedded in the line chart based on supplemental information. The one or more indicators may vary responsively to the first data samples collected at each of the sampling times. The supplemental information may also include second data samples of ancillary data with respect to the patient and/or a surgical procedure collected at the sampling times.

### BRIEF DESCRIPTION OF THE DRAWINGS

A more detailed understanding may be had from the following description, given by way of example in conjunction with the accompanying drawings wherein:
FIG. 1 is a schematic, pictorial illustration of a medical system configured to present an enhanced electrocardiography (ECG) chart;
FIG. 2 is a schematic view showing a distal tip of a catheter in contact with endocardial tissue of a cardiac chamber;
FIG. 3 is a flow diagram that schematically illustrates a method of presenting the ECG chart;
FIG. 4 is a flow diagram that schematically illustrates a method of presenting the ECG chart that includes second data samples, in accordance with an embodiment of the present invention;
FIG. 5 is a schematic view of an enhanced ECG chart;
FIGS. 6A-6D are diagrams illustrating color coding schemes that may be embedded in the enhanced ECG chart to indicate different types of supplemental information; and
FIG. 7 is a schematic view of another enhanced ECG chart.

### DETAILED DESCRIPTION

Documents incorporated by reference in the present patent application may include terms that are defined in a manner that conflicts with the definitions made explicitly or implicitly in the present specification. In the event of any conflicts, the definitions in the present specification should be considered to be controlling.

The following description relates generally to electrocardiography (ECG), and more specifically to methods, systems, and apparatuses that present ECG data as well as ancillary electrophysiological data and other patient data in a single chart.

During a medical procedure such as cardiac ablation, there are typically simultaneous streams of real-time data that an operator (e.g., a physician) monitors while performing the procedure. For example, while using an intracardiac catheter to perform an ablation on intracardiac tissue, the operator may want to monitor real-time electrophysiological (EP) data such as electrocardiography (ECG) data, and ancillary data such as locations of the distal tip of the catheter and ablation energy being delivered to the heart tissue. In some procedures, there may be a need to show information which is interpreted or deciphered from the signal such as timing between consecutive activations and dominant frequency.

The operator may need to be aware of many real-time indicators located in signals shown in different areas of a display. Typically, these indicators may be values of different types of ancillary data, or a relative change of these values. With the various different indicators being presented, the operator may be burdened by tracking multiple sources of information simultaneously. It may be desirable to consolidate some of the information into a unified view presented on top of an electrocardiography (ECG) signal (e.g., body surface and intracardiac) in real-time and display them as an enhanced ECG chart. The enhanced ECG chart may enable the operator to remain focused on the modified signal that includes the embedded indicators being transmitted in real time instead of switching focus between the different areas on the display, such as different views, pages, and/or tabs, or even different monitors.

In a medical procedure, such as cardiac ablation on cardiac tissue, the ancillary data may include measurements received from a distal end of an intracardiac catheter within a cardiac chamber. Examples of these measurements may include, but are not limited to, force, tissue proximity, temperature of intracardiac tissue, positions of the distal end, respiration indicators, local activation time (LAT) values, and measurements of ablation energy delivered by the distal end of the catheter to the intracardiac tissue.

The ECG data may be presented as a chart (e.g., a line chart) on the display. The ancillary data may be presented to the operator by embedding a visual representation of the values of the measurements, or relative changes in the values of the measurements, into the ECG chart. By combining the ECG data and the ancillary data into a single chart, an operator may be able to track multiple ECG and ancillary data parameters by looking at the single chart.

Upon collecting first data samples of electrical potentials produced by a heart at a sequence of sampling times, the first data samples are presented as an ECG chart on a display. The ECG chart may be a trace of the electrical potentials collected at the sampling times. In addition to collecting the first data, second data samples of the ancillary data may also be collected at the sampling times. As described in additional detail below, supplemental information, such as cycle length (CL) stability and/or CL variability, may be calculated from the first data samples. The supplemental information may also include the second data samples. The supplemental information may be presented as an embedded trace on the ECG chart that varies responsively to the ancillary data collected at each of the sampling times.

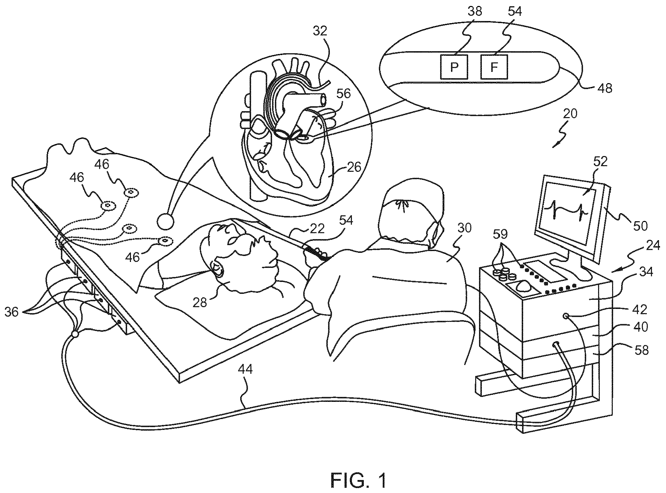

Referring now to FIG. 1, an illustration of a medical system 20 that may be used to generate and display a chart 52 is shown. The system 20 may include a probe 22, such as an intracardiac catheter, and a console 24. As described herein, it may be understood that the probe 22 is used for diagnostic or therapeutic treatment, such as for mapping electrical potentials in a heart 26 of a patient 28. Alternatively, the probe 22 may be used, mutatis mutandis, for other therapeutic and/or diagnostic purposes in the heart, lungs, or in other body organs and ear, nose, and throat (ENT) procedures.

An operator 30 may insert the probe 22 into the vascular system of the patient 28 so that a distal end 32 of the probe 22 enters a chamber of the patient's heart 26. The console 24 may use magnetic position sensing to determine position coordinates of the distal end 32 inside the heart 26. To determine the position coordinates, a driver circuit 34 in the console 24 may drive field generators 36 to generate magnetic fields within the body of the patient 28. The field generators 36 may include coils that may be placed below the torso of the patient 28 at known positions external to the patient 28. These coils may generate magnetic fields in a predefined working volume that contains the heart 26.

A location sensor 38 within the distal end 32 of probe 22 may generate electrical signals in response to these magnetic fields. A signal processor 40 may process these signals in order to determine the position coordinates of the distal end 32, including both location and orientation coordinates. The method of position sensing described hereinabove is implemented in the CARTO™ mapping system produced by Biosense Webster Inc., of Diamond Bar, Calif., and is described in detail in the patents and the patent applications cited herein.

The location sensor 38 may transmit a signal to the console 24 that is indicative of the location coordinates of the distal end 32. The location sensor 38 may include one or more miniature coils, and typically may include multiple coils oriented along different axes. Alternatively, the location sensor 38 may comprise either another type of magnetic sensor, or position transducers of other types, such as impedance-based or ultrasonic location sensors. Although FIG. 1 shows the probe 22 with a single location sensor 38, embodiments of the present invention may utilize probes without a location sensor 38 and probes with more than one location sensor 38.

The probe 22 may also include a force sensor 54 contained within the distal end 32. The force sensor 54 may measure a force applied by the distal end 32 to the endocardial tissue of the heart 26 and generating a signal that is sent to the console 24. The force sensor 54 may include a magnetic field transmitter and a receiver connected by a spring in the distal end 32, and may generate an indication of the force based on measuring a deflection of the spring. Further details of this sort of probe and force sensor are described in U.S. Patent Application Publications 2009/0093806 and 2009/0138007, whose disclosures are incorporated herein by reference. Alternatively, the distal end 32 may include another type of force sensor that may use, for example, fiber optics or impedance measurements.

The probe 22 may include an electrode 48 coupled to the distal end 32 and configured to function as an impedance-based position transducer. Additionally or alternatively, the electrode 48 may be configured to measure a certain physiological property, for example the local surface electrical potential of the cardiac tissue at one or more of the multiple locations. The electrode 48 may be configured to apply radio frequency (RF) energy to ablate endocardial tissue in the heart 26.

Although the example medical system 20 may be configured to measure the position of the distal end 32 using magnetic-based sensors, other position tracking techniques may be used (e.g., impedance-based sensors). Magnetic position tracking techniques are described, for example, in U.S. Pat. Nos. 5,391,199, 5,443,489, 6,788,967, 6,690,963, 5,558,091, 6,172,499, and 6,177,792, whose disclosures are incorporated herein by reference. Impedance-based position tracking techniques are described, for example, in U.S. Pat. Nos. 5,983,126, 6,456,8208 and 5,944,022, whose disclosures are incorporated herein by reference.

The signal processor 40 may be included in a general-purpose computer, with a suitable front end and interface circuits for receiving signals from the probe 22 and controlling the other components of the console 24. The signal processor 40 may be programmed, using software, to carry out the functions that are described herein. The software may be downloaded to the console 24 in electronic form, over a network, for example, or it may be provided on non-transitory tangible media, such as optical, magnetic or electronic memory media. Alternatively, some or all of the functions of the signal processor 40 may be performed by dedicated or programmable digital hardware components.

In the example of FIG. 1, the console 24 may also be connected by a cable 44 to external sensors 46. The external sensors 46 may include body surface electrodes and/or position sensors that may be attached to the patient's skin using, for example, adhesive patches. The body surface electrodes may detect electrical impulses generated by the polarization and depolarization of cardiac tissue. The position sensors may use advanced catheter location and/or magnetic location sensors to locate the probe 22 during use. Although not shown in FIG. 1, the external sensors 46 may be embedded in a vest that is configured to be worn by the patient 28. The external sensors 46 may help identify and track the respiration cycle of the patient 28. The external sensors 46 may transmit information to the console 24 via the cable 44.

Additionally, or alternatively, the probe 22, and the external sensors 46 may communicate with the console 24 and one another via a wireless interface. For example, U.S. Pat. No. 6,266,551, whose disclosure is incorporated herein by reference, describes, inter alia, a wireless catheter, which is not physically connected to signal processing and/or computing apparatus. Rather, a transmitter/receiver may be attached to the proximal end of the probe 22. The transmitter/receiver communicates with a signal processing and/or computer apparatus using wireless communication methods, such as infrared (IR), radio frequency (RF), wireless, Bluetooth, or acoustic transmissions.

The probe 22 may be equipped with a wireless digital interface (not shown) that may communicate with a corresponding input/output (I/O) interface 42 in the console 24. The wireless digital interface and the I/O interface 42 may operate in accordance with any suitable wireless communication standard that is known in the art, such as IR, RF, Bluetooth, one of the IEEE 802.11 families of standards, or the HiperLAN standard. The external sensors 46 may include one or more wireless sensor nodes integrated on a flexible substrate. The one or more wireless sensor nodes may include a wireless transmit/receive unit (WTRU) enabling local digital signal processing, a radio link, and a power supply such as miniaturized rechargeable battery.

The I/O interface 42 may enable the console 24 to interact with the probe 22 and the external sensors 46. Based on the electrical impulses received from the external sensors 46 and signals received from the probe 22 via the I/O interface 42 and other components of the medical system 20, the signal processor 40 may generate the chart 52, which may be shown on a display 50.

During the diagnostic treatment, the signal processor 40 may present the chart 52 and may store data representing the chart 52 in a memory 58. The memory 58 may include any suitable volatile and/or non-volatile memory, such as random access memory or a hard disk drive. The operator 30 may be able to manipulate the chart 52 using one or more input devices 59. Alternatively, the medical system 20 may include a second operator that manipulates the console 24 while the operator 30 manipulates the probe 22.

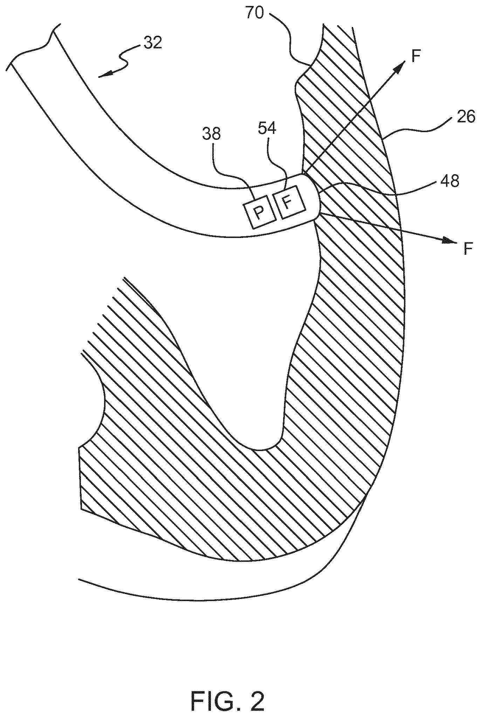

Referring now to FIG. 2, a schematic detail view illustrating the distal end 32 of the probe 22 in contact with endocardial tissue 70 of the heart 26 is shown. As described above, the operator 30 may advance the probe 22 so that the distal end 32 engages endocardial tissue 70 and exerts force F on the endocardial tissue.

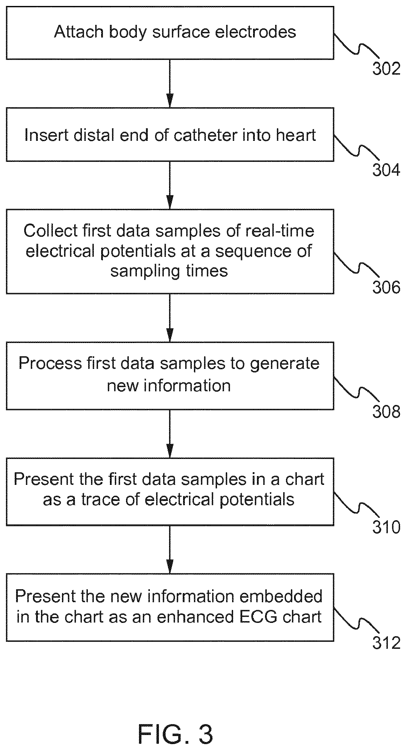

Referring to FIG. 3, a flow diagram illustrating an overview of a method for presenting the enhanced ECG chart 52 showing ECG data and supplemental information collected during a procedure on the heart 26 is shown. The flow diagram of FIG. 3 may be best understood in conjunction with a diagram illustrating the distal end 32 of the probe 22 in contact with endocardial tissue 70 of the heart 26 as shown in FIG. 2.

In an initial step 302, the operator 30 may attach the external sensors 46 to the patient 28. As described above, the external sensors 46 may include body surface electrodes and/or position sensors that may be attached to the patient's skin or embedded in a vest. In step 304, the operator 30 may insert the probe 22 into a chamber of the heart 26, which may be referred to herein as the cardiac chamber.

In a first collection step 306, first data samples including electrical potentials produced by the heart 26 at a sequence of sampling times may be collected. The sequence of sampling times may be discreet time points at which the electrical potential is measured. The sampling times may occur periodically, for example, approximately every 0.125ms. The sequence of sample times may occur over one or more cycles of cardiac rhythms.

The first data samples may be gathered by the electrode 48 coupled to the distal end 32 of the probe 22 and may be considered an intra-cardiac electrocardiogram (ECG). Additionally, or alternatively, the first data samples may be gathered by the external sensors and may be considered an inter-cardiac ECG. The first data samples may be gathered in real time and may be sent to the signal processor 40 as described above.

In step 308, the first data samples may be processed by the signal processor 40 to generate supplemental information. The signal processor 40 may accumulate a number of the first data samples over a period of multiple sampling times and use them to calculate the supplemental information. For example, the signal processor 40 may use the first data samples to calculate a real time cycle length (CL) stability value. In this context, the cycle length is the time difference between two consecutive activations on one ECG channel. The CL stability may be calculated by determining the difference between the last CL measurement and the previous measured CL. Alternatively, the CL stability may be calculated by determining the difference between the last CL measured and an average CL of a predetermined and configurable number of previous CLs. Other examples of supplemental information that may be calculated include CL variation, timing differences between consecutive activations, stability of the timing differences between activations, and dominant frequency.

The variation in CL may be calculated by one or more of the following methods. The CL variation may be calculated by determining the CL over a number of consecutive annotations. An average CL of these annotations may be established. The CL variation may be considered as the difference between the average CL value and each individual CL value. It should be noted that the number of consecutive annotations used to establish the average may vary depending on the application.

The CL variation may be calculated by determining the CL from one or more consecutive annotations. The determined CL may then be compared to a measured CL of a next consecutive annotation. The difference between these values may be used to determine CL variation and/or CL stability.

The CL variation may be calculating by determining the CL over a number of consecutive annotations and establishing a dominant (mean) CL of these annotations. The difference between the dominant CL value and each independent CL value may be used to determine CL variation and/or CL stability. The number of consecutive annotations used to establish the average or mean CL may vary depending on the application.

The dominant frequency may be determined using frequency domain analysis. The first data samples (i.e., the ECG information) may be processed and segmented into, discrete windows of a predetermined length (e.g., four seconds) with a predetermined overlap (e.g., three seconds).

A periodogram of the segmented first data samples may be generated. The periodogram may be used to determine the significance of different frequencies in the segmented first data samples to identify intrinsic periodic signals. The periodogram may be multiplied by a Hanning window. The windowing procedure may gradually attenuate discontinuities at a beginning and end of a time segment to zero in order to lessen their effect on a final spectrum. The dominant frequency may be extracted as a maximum value of the final spectrum.

The dominant frequency may also be calculated using a pwelch approach. The segmented first data samples may be further segmented. For example, the four second windows may be segmented an additional 8 times with a 50% overlap (i.e., 1 second). Periodograms of the 8 segments may be averaged in order to generate a final spectrum. The dominant frequency may be extracted as a maximum value of the final spectrum.

To ensure reliability in the detection of the dominant frequency, a regularity index may be calculated as the ratio of the power at the dominant frequency and its adjacent frequencies to the power of the 2.5 to 20 Hz band. Points demonstrating a regularity index above 0.2 and a deviation of less than 0.5 Hz from the dominant frequency estimated by the methods described above may be included in subsequent analyses to control for ambiguity in dominant frequency detection.

In step 310, the first data samples may be presented in a chart as a trace of the collected electrical potentials. The trace chart of collected electrical potentials may include a first line that plots potentials along a vertical axis against time along a horizontal axis, wherein the potentials are measured as voltages V and the time is measured in seconds S.

In step 312, the supplemental information may be embedded into the trace chart to create the enhanced ECG chart 52. The supplemental information may be combined with the trace chart, such that the supplemental information is presented on the trace chart with different a color, shading, or thickness to indicate different values. The supplemental information may be superimposed over the trace chart at continuous or discreet time points. The supplemental information may be displayed as data points embedded into the trace chart. The supplemental information may be presented in real time as the first data samples are gathered. The enhanced ECG chart 52 may be described in further detail below. The signal processor 40 may save the first data samples and the supplemental information to the memory 58.

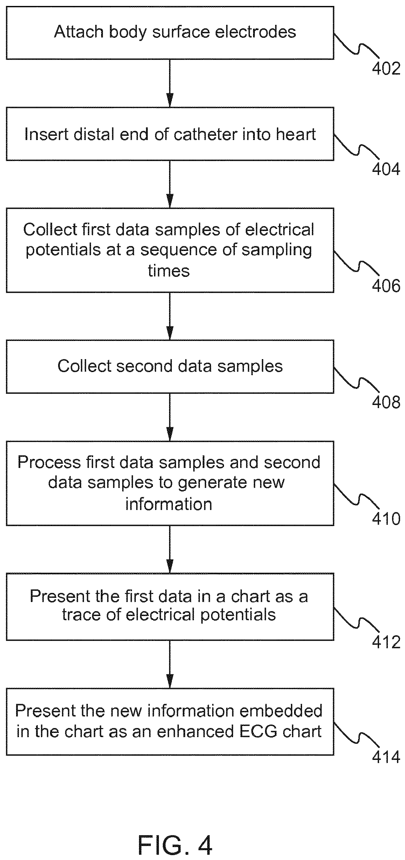

Referring now to FIG. 4, a flow diagram illustrating an overview of a method for presenting the enhanced ECG chart 52 showing ECG data and supplemental information containing second data samples collected during a procedure on the heart 26 is shown. The flow diagram of FIG. 4 may be best understood in conjunction with a diagram illustrating the distal end 32 of the probe 22 in contact with endocardial tissue 70 of the heart 26 as shown in FIG. 2.

In an initial step 402, the operator 30 may attach the external sensors 46 to the patient 28. As described above, the external sensors 46 may include body surface electrodes and/or position sensors that may be attached to the patient's skin or embedded in a vest. In step 404, the operator 30 may insert the probe 22 into a chamber of the heart 26, which may be referred to herein as the cardiac chamber.

In a first collection step 406, first data samples including electrical potentials produced by the heart 26 at a sequence of sampling times may be collected. The sequence of sampling times may be discreet time points at which the electrical potential is measured. The sampling times may occur periodically, for example, approximately every 0.125ms. The sequence of sample times may occur over one or more cycles of cardiac rhythms.

The first data samples may be gathered by the electrode 48 coupled to the distal end 32 of the probe 22 and may be considered an intracardiac electrocardiogram (ECG). Additionally, or alternatively, the first data samples may be gathered by the external sensors and may be considered an intercardiac ECG. The first data samples may be gathered in real time and may be sent to the signal processor 40 as described above.

In step 408, second data samples may be collected with respect to the patient 28 and the heart 26. The second data samples may be collected simultaneously with the first data samples at the sampling times. The second data samples may include measurements received from one or more sensors mounted in the distal end 32 of the probe 22. For example, as the operator 30 advances the probe 22 so that the distal end 32 engages the endocardial tissue 70 and exerts a force "F" on the endocardial tissue, the second data samples may comprise force measurements received from the force sensor 54 that indicate force F.

Additional examples of second data samples that the signal processor 40 may receive from the probe 22 or other elements of the console 24 may include, but are not limited to the following measurements. One example may be a magnitude and phase of an impedance detected by the surface electrodes in the external sensors 46. Another example may be a position of the distal end 32. The position signals received from the location sensor 38 may indicate a distance between the distal end 32 and the endocardial tissue 70.

Another example may be a quality of contact between the distal end 32 and the endocardial tissue 70, as indicated by force signals received from the force sensor 54. The quality of contact may include a magnitude and a direction of force F. Another example may be a measurement of ablation energy delivered by the electrode 48 to endocardial tissue. Typically, the ablation energy varies during an ablation procedure.

Another example may be starting and ending times indicating when ablation energy is delivered to the endocardial tissue. Another example may be irrigation parameters such as starting and ending times, indicating when the probe 22 is delivering irrigation fluid to the endocardial tissue 70, as well as pressures and temperatures of the irrigation fluid.

Another example may be a temperature of the endocardial tissue in contact with the distal tip. Another example may be a Force Power Time Integral (FPTI). The FPTI may be a scalar value that represents the force power time integral during ablation. During an ablation procedure, the FPTI value indicates a quality of an ablation lesion.

In step 410, the first data samples and the second data samples may be processed by the signal processor 40 to generate supplemental information. The signal processor 40 may accumulate a number of the first data samples over a period of multiple sampling times and use them to calculate the supplemental information. Examples of the supplemental information that may be generated from the first data samples are described above with reference to FIG. 3. Additionally or alternatively, the supplemental information may be based on the measurement values of the second data samples.

In step 412, the first data samples may be presented in a chart as a trace of the collected electrical potentials. The trace chart of collected electrical potentials may include a first line that plots potentials along a vertical axis against time along a horizontal axis, wherein the potentials are measured as voltages V and the time is measured in seconds S.

In step 414, the supplemental information may be embedded into the trace chart to create the enhanced ECG chart 52. The supplemental information may be combined with the trace chart, such that the supplemental information is presented on the trace chart with different a color, shading, or thickness to indicate different values. The supplemental information may be superimposed over the trace chart at continuous or discreet time points. The supplemental information may be displayed as data points embedded into the trace chart. The supplemental information may be presented in real time as the first data samples are gathered. The embedded characteristics may vary responsively to the second data samples collected at each of the sampling times. The enhanced ECG chart 52 may be described in further detail below. The signal processor 40 may save the first data samples, the second data samples, and the supplemental information to the memory 58.

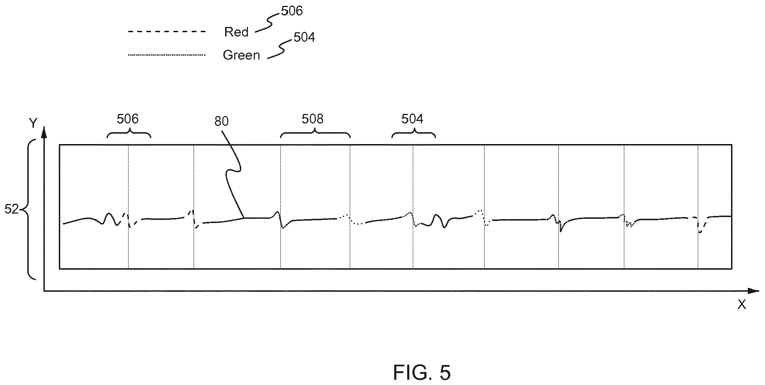

Referring now to FIG. 5, a diagram illustrating an enhanced ECG chart 52 is shown. The signal processor 40 may present the enhanced ECG chart 52 as a line chart with areas having different colors, thicknesses, and data points representing the supplemental information as an easily readable form embedded in the trace chart. The enhanced ECG chart 52 may include a line 80 that plots potentials along a vertical axis y against time along a horizontal axis x, wherein the potentials are measured as voltages V and the time is measured in seconds S.

One or more items of information embedded in an ECG signal may have a selectable second real time stream of data superimposed on the first real time stream of data. For example, as an item of the ECG signal is displayed in real time, the real time CL stability may be embedded onto the signal. Cycle instability may be shown as a sinusoidal wave. In one embodiment, the supplemental information may be embedded by superimposing the data onto the ECG signal. In another embodiment, the supplemental information may be displayed in a different color. In yet another embodiment, the supplemental information may be displayed as data points embedded onto the ECG signal.

The signal processor 40 may vary the color, shading, and thickness of the enhanced ECG chart 52 in order to indicate values of the supplemental information. For example, as the operator 30 presses the distal end 32 against the endocardial tissue 70, the signal processor 40 may vary the color of the line 80 from green 504 to represent less force to red 506 to represent more force based on the force F. In another example, when real time CL stability data is embedded, red 506 may indicate lower stability and green 504 may indicate higher stability. In another example, the signal processor 40 may vary the color of the line 80 in order to indicate a distance between distal end 32 of the probe 22 and the endocardial tissue 70. For example, the signal processor 40 can change the color of the line from green 504 to red 506 as the distal end 32 moves closer to and engages endocardial tissue 70.

The color coding may be used in one or more annotations on the enhanced ECG chart 52. The one or more annotations may serve as a marker in that signifies an important moment for the operator 30. The color coding and the annotation may occur once every cardiac cycle 508, which may be indicated by vertical lines in FIG. 5. The cardiac cycle rate may vary depending on the condition of the patient. The length of each color coding segment along the one or more annotations may be long enough for the operator 30 to notice but short enough not to merge with another segment. Additionally, or alternatively, the signal processor 40 may vary the thickness of the enhanced ECG chart 52 in order to indicate the values of the second data samples.

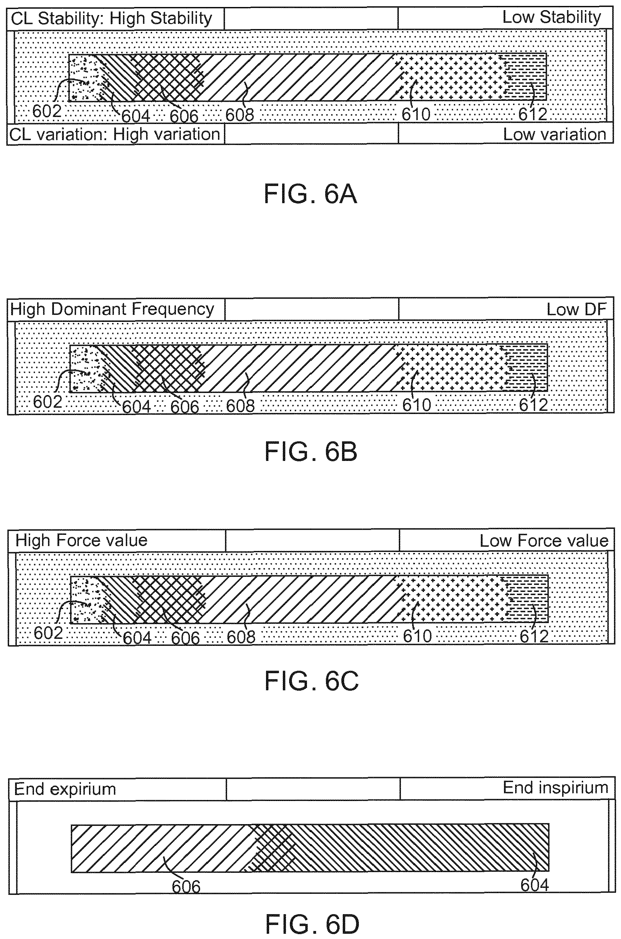

Referring now to FIGS. 6A-6D, diagrams illustrating color coding schemes that may be embedded in the enhanced ECG chart 52 to indicate different types of supplemental information are shown. It should be noted that although the figures are shown in greyscale, embodiments may use the full color spectrum visible to the human eye.

FIG. 6A illustrates a color coding scheme that may indicate CL stability and/or CL variation. On one end of a continuous color spectrum (e.g., ranging from red 602, orange 604, yellow 606, green 608, blue 610, and violet 612), a red color 602 may indicate a high CL stability. On the other end of the continuous color spectrum, a violet color 612 may indicate a low CL stability. In addition, on one end of the continuous color spectrum, the red color 602 may indicate a high CL variation. On the other end of the continuous color spectrum, a violet color 612 may indicate a low CL variation.

FIG. 6B illustrates a color coding scheme that may indicate dominant frequency. On one end of a continuous color spectrum (e.g., ranging from red 602, orange 604, yellow 606, green 608, blue 610, and violet 612), a red color 602 may indicate a high dominant frequency. On the other end of the continuous color spectrum, a violet color 612 may indicate a low dominant frequency.

FIG. 6C illustrates a color coding scheme that may indicate force of the probe 22 on cardiac tissue. As described above, the force value may be provided by one or more sensors on the distal end 32 of the probe 22. On one end of a continuous color spectrum (e.g., ranging from red 602, orange 604, yellow 606, green 608, blue 610, and violet 612), a red color 602 may indicate a high force value. On the other end of the continuous color spectrum, a violet color 612 may indicate a low force value.

FIG. 6D illustrates a color coding scheme that may indicate a respiration cycle. As described above, the one or more external sensors 46 may track chest movement to determine respiration cycles. On one end of a continuous color spectrum between two colors (e.g., ranging from yellow 606 to orange 604), a yellow color 606 may indicate an end of expirium. On the other end of the continuous color spectrum, an orange color 604 may indicate an end of inspirium.

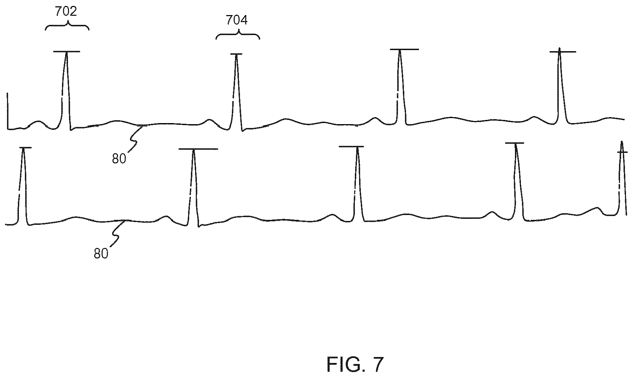

Referring to FIG. 7, a diagram illustrating another enhanced ECG chart 52 is shown. Instead of using the color coding scheme described above to indicate different values of the supplemental information, the supplemental information may be presented as a series of horizontal lines above the line 80 on the trace chart. The horizontal lines may be included above each annotation. Different values of the supplemental information may be represented by different lengths of the horizontal lines. For example, larger values (e.g., a high force value) may be indicated by longer lines 702 and smaller values (e.g., a low force value) may be indicated by shorter lines 704.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

## Claims

1. A method comprising:
collecting first data samples of electrical potentials produced by a heart at a sequence of sampling times, wherein the first data samples are collected by one or more electrodes;
generating supplemental information based on at least the differences in the first data samples gathered over a number of the sampling times;
generating, based on the first data samples, a trace of the electrical potentials collected at the sampling times, wherein the trace comprises a line chart; and
embedding one or more indicators in the line chart based on the supplemental information, wherein the one or more indicators vary responsively to the first data samples collected at each of the sampling times.

2. The method of claim 1, further comprising:
collecting second data samples with respect to the heart at the sampling times; and
displaying the second data samples as the one or more embedded indicators.

3. The method of claim 1, further comprising:
generating an icon on the line chart indicating an occurrence of one or more events; and
providing information on the one or more events upon receiving an input selecting the icon.

4. The method of claim 1, wherein the embedding one or more indicators in the line chart occurs in real time.

5. An apparatus, comprising:
a console having one or more processors; and
a non-transitory computer readable medium storing a plurality of instructions, which when executed, cause the one or more processors to:
collect first data samples of electrical potentials produced by a heart at a sequence of sampling times, wherein the first data samples are collected by one or more electrodes,
generate supplemental information based on at least the differences in the first data samples gathered over a number of the sampling times;
generate, based on the first data samples, a trace of the electrical potentials collected at the sampling times, wherein the trace comprises a line chart; and
embed one or more indicators in the line chart based on the supplemental information, wherein the one or more indicators vary responsively to the first data samples collected at each of the sampling times.

6. The method of claim 1 or the apparatus of claim 5, wherein the one or more electrodes are located on a distal end of a probe inserted into the heart.

7. The method of claim 1 or the apparatus of claim 5, wherein the one or more electrodes are external to the heart.

8. The apparatus of claim 5, wherein the plurality of instructions, when executed, further cause the one or more processors to collect second data samples and display the second data samples as the one or more embedded indicators.

9. The method of claim 2 or the apparatus of claim 8, wherein the second data samples comprise measurements selected from a list consisting of ablation energy, a location of the distal end of the flexible probe, a measurement of a force exerted by the distal end on endocardial tissue of the heart, a quality of contact between the distal end and the endocardial tissue, a magnitude and a phase of impedance detected by the body surface electrodes, a temperature of the endocardial tissue, a Force Power Time Integral, irrigation fluid parameters, S-Waves, noise level, and respiratory indication.

10. The method of claim 1 or the apparatus of claim 5, wherein the supplemental information comprises metrics of the electrical potentials measured over time such as real time cycle length stability.

11. The method of claim 1 or apparatus of claim 5, wherein the one or more embedded indicators represent a relative change in value of the supplemental information.

12. The method of claim 1 or apparatus of claim 5, wherein the line chart has a vertical axis comprising values of the first data samples and a horizontal axis comprising time.

13. The apparatus of claim 5, wherein the instructions, which when executed, further cause the one or more processors to:
generate an icon on the line chart indicating an occurrence of one or more events, and
provide information on the one or more events upon receiving an input selecting the icon.

14. The apparatus of claim 5, wherein the plurality of instructions, when executed, further cause the one or more processors to:
embed the one or more indicators in the line chart in real time.
